# EUROPÄISCHE PATENTSCHRIFT

(11) **EP 2 708 900 B1**
(45) Veröffentlichungstag und Bekanntmachung des Hinweises auf die Patenterteilung: **12.04.2017**
(21) Anmeldenummer: 13196301.9
(22) Anmeldetag: 09.09.2010
(51) Int. Cl.: G01N 33/86, G01N 29/036

(54) **Verfahren zur Bestimmung der Thrombozytenfunktion mit einem Resonator**
Method for determining thrombocyte functions by means of a resonator
Procédé de détermination de la fonction de thrombocytes au moyen d'un résonateur

(30) Priorität: 09.09.2009 DE 102009040881
(43) Veröffentlichungstag der Anmeldung: 19.03.2014
(62) Teilanmeldung aus: 10760912.5
(73) Patentinhaber: Andreas Hettich GmbH & Co. KG, D-78532 Tuttlingen (DE)
(72) Erfinder: Gehring, Frank K., 72364 Obernheim (DE); Wendel, Hans-Peter, 72336 Balingen (DE); Sinn, Stefan, 71083 Herrenberg (DE); Müller, Lothar, 65197 Wiesbaden (DE)
(74) Vertreter: Puschmann Borchert Bardehle Patentanwälte Partnerschaft mbB

(56) Entgegenhaltungen:
- TAKEHISA MATSUDA ET AL: "NOVEL INSTRUMENTATION MONITORING IN SITU PLATELET ADHESIVITY WITH A QUARTZ CRYSTAL MICROBALANCE", ASAIO JOURNAL, LIPPINCOTT WILLIAMS & WILKINS / ASAIO, HAGERSTOWN, MD, US, Bd. 38, Nr. 3, Juli 1992 (1992-07), Seiten 171-173, XP000321538, ISSN: 1058-2916

## Beschreibung

Die Erfindung betrifft ein Verfahren zur Bestimmung der Thrombozytenfunktion gemäß der im Oberbegriff des Anspruchs 1 angegebenen Art.

Für die Blutgerinnung müssen sowohl die primäre als auch die sekundäre Hämostase funktionsfähig sein. Zur Überprüfung der sekundären Hämostase sind viele Möglichkeiten bekannt. Für die primäre Hämostase, die sich in Adhäsion und Aggregation von Thrombozyten unterteilt, ist dem Stand der Technik kein ausreichend genaues Analyseverfahren zur Bestimmung der spezifischen Thrombozytenfunktion zu entnehmen.

Nach dem Stand der Technik sind Blutanalysen, welche die Hämostase betreffen möglich. Jedoch ist eine Unterscheidung, bei der primären Hämostase, ob Adhäsion oder Aggregation geschädigt ist, oder in welcher Art die Aggregation gehemmt ist, nicht möglich.

Es ist bekannt, dass Blutplättchen auf der Oberfläche eines Schwingquarzes durch Adhäsion angelagert werden können. Diese Anlagerung ist qualitativ messbar, lässt jedoch keine spezifische Identifikation einer Fehlfunktion der Aggregation zu.

Die Veröffentlichung "real-time monitoring of adhesion and aggregation of platelets using thickness shear mode sensors" (E. Ergezen et al., Biosensors and Bioelectronics 23 (2007), 575 - 582) beschreibt eine Analyse der Blutgerinnung von Adhäsion und Aggregation von Blutplättchen mittels eines Schwingquarzes. Diese Schrift zeigt, dass verschiedene Phasen, Adhäsion, und Aggregation mit Hilfe eines Schwingquarzes qualitativ nachweisbar ist. Sie löst nicht das Problem, die Funktionsfähigkeit der Thrombozyten zu Adhäsion und Aggregation zu unterscheiden. Aus dieser Druckschrift ist bekannt, dass die Aggregation von mangelnder Adhäsionsfähigkeit gut abgrenzbar ist, da eine erfolgreiche Aggregation eine hohe Frequenz bzw. Dämpfungsänderung nach sich zieht.

Entsprechend ist bekannt die Aggregationsfähigkeit festzustellen. Zwischen Adhäsionsfähigkeit und Aggregationsfähigkeit der Thrombozyten kann nach diesem Verfahren nicht zuverlässig unterschieden werden, da der Frequenzhub nicht nur von der Zugabe von ADP abhängig ist, sondern auch von der Thrombozytenkonzentration, der Bindungsstärke der Schwingquarzoberfläche und der Adhäsionsfähigkeit der Thrombozyten. Durch diese Betrachtung kann möglicherweise eine falschpositive Entscheidung erfolgen, da lediglich zwischen einem erfolgten Frequenzabfall und keinem Frequenzabfall unterschieden wird, wobei auch lediglich die Adhäsion der Thrombozyten unter bestimmten Vorraussetzungen zu einem starken Frequenzabfall führen kann. Auch die US 2005/0015001 A1 beschreibt im Wesentlichen das oben genannte.

Die Druckschrift "A new method for continuous measurement of platelet adhesion under flow conditions" (K. Kawakami et al., ASAIO Journal 39 (1993), M558 - M560) offenbart, dass eine Adhäsion von Plättchen messbar ist, stellt aber keinen Bezug zur Analyse einer Dysfunktion noch einer Kombination mit einer Bewertung der Aggregationsfunktion her.

Im Vortrag "Potenzial der Schwingquarzsensoren zur inline Hämothese-Prüfung" (F.K. Gehring, Miniaturisierte Biosystemtechnik, BMBF, Hannover, 07.10.2008) auf Folie 24 sind zwei Frequenzverläufe dargestellt, die Rückschluss auf die Thrombozytenfunktion geben sollen. Eine Darstellung zeigt einen Frequenzabfall bei einer Schwingquarzmessung von aktiviertem Blut, die zweite Kurve zeigt keinen Frequenzabfall bei inhibiertem Blut. Dies gibt in der Form lediglich wieder, dass sich eine Funktion anhand des Verhaltens der Schwingungsparameter ablesen lässt. Es ist aufgrund dieser Unterlagen unklar, ob die dargelegte Funktion eine Adhäsion, Aggregation oder sonstige Ablagerung eines Stoffs auf der Schwingquarzoberfläche abbildet. Eine eindeutige Funktionszuordnung kann dieser Offenbarung nicht entnommen werden.

Auch die Druckschrift "Novel instrumentation monitoring in situ platelet adhesivity with a quartz crystal microbalance" (T. Matsuda, ASAIO 38 (1992), M171 - M173) beschreibt eine Untersuchung der Thrombozytenfunktion mittels eines Schwingquarzes, wobei der zeitliche Verlauf der Resonanzfrequenz beobachtet wird.

Es ist die Aufgabe der Erfindung ein Verfahren anzugeben mittels dem eine Aussage getroffen werden kann, welcher Zweig der zellulär vermittelten Gerinnung gestört ist.

Die Aufgabe wird durch die kennzeichnenden Merkmale des Anspruchs 1 gelöst.

In bekannter Weise werden zur Bestimmung von Parametern der primären Hämostase die Schwingungsparameter eines Resonators gemessen, der mit einer Oberfläche versehen ist, die in Kontakt mit einem Thrombozyten enthaltenden Probenfluid steht. Dadurch lagern sich Thrombozyten oder andere Blutbestandteile an der Oberfläche des Schwingquarzes an, die dessen Schwingungsverhalten beeinflussen.

Erfindungsgemäß wird zur Bestimmung der Thrombozytenfunktion ein Probenfluid auf eine Resonatoroberfläche und eine Analyse der Charakteristik beziehungsweise Kinetik der oder des gemessenen Schwingungsparameters nämlich der Dämpfung bzw. Amplitude des Resonators durchgeführt, aufgrund der die Thrombozytenfunktion bewertet wird. Anhand der Charakteristik wird zwischen totaler Dysfunktion bzw. Adhäsionsstörung und Aggregationsstörung der Thrombozyten unterschieden. Es wird die Charakteristik des Verlaufs der Schwingungsparameter über die Zeit ausgewertet und abhängig davon wird eine Aussage getroffen, welcher Zweig der zellulär vermittelten Gerinnung gestört ist. Entspricht das Schwingungsverhalten des Schwingquarzes mit aktivierter Probe dem der nicht aktivierten Probe, so wird darauf geschlossen, dass die Thrombozyten adhäsionsfähig aber nicht aggregationsfähig sind. Es wird auf eine Aggregationsstörung geschlossen. Verändert sich die Kurve der Schwingungsparameter über die Zeit nicht, wird auf eine Dysfunktion in der Thrombozytenfunktion geschlossen. Dies bedeutet, dass die Thrombozyten nicht adhärieren können, was zur Folge hat, dass auch keine Aggregation möglich ist.

Durch dieses Verfahren wird eine Diagnostik mit einem Resonator ermöglicht.

Dies hat den enormen Vorteil, dass durch eine Unterscheidung welcher Zweig der Gerinnung gestört ist eine entsprechend abgestimmte Medikation erfolgen kann. Grundsätzlich wird aufgrund dieser Untersuchungsmöglichkeiten ein großer informationspool der zellulär vermittelten Gerinnung bereitgestellt. Eine gezielte Medikation vermindert die Nebenwirkungen für den Patienten deutlich und kann bedeutend Kosten sparen.

Grundsätzlich werden in einer ersten Ausführungsform die Schwingungsparameter in Form der Resonanzfrequenz ausgewertet. Alternativ dazu kann auch die Dämpfung bzw. Amplitude des Resonators ausgewertet werden. Insbesondere wird die Grundschwingung betrachtet.

Nach Starten der Messung wird ein Thrombozyten enthaltendes Probefluid auf den Schwingquarz aufgebracht. Verändert sich die Kurve der Schwingungsparameter über die Zeit nicht, wird auf eine Dysfunktion in der Thrombozytenfunktion geschlossen. Dies bedeutet, dass die Thrombozyten nicht adhärieren können, was zur Folge hat, dass auch keine Aggregation möglich ist. Eine weitere Untersuchung ist nicht notwendig, da sofern keine Adhäsion möglich ist auch keine Blutgerinnung erfolgt. Die Dysfunktion entspricht also einer Adhäsionsstörung.

Im Gegensatz dazu schließt man bei einem mehr oder weniger linearen Abfall der Schwingungsparameter über die Zeit auf eine grundsätzliche Adhäsionsfähigkeit der Thrombozyten. Die Adhäsion kann durch Zusatz eines Inhibitors verhindert werden. So kann beispielsweise der GPIIb/IIIa - Rezeptor blockiert werden, um eine Adhäsion zu vermeiden. Es ist also möglich, dass nachdem eine Probe positiv auf ihre Adhäsionsfunktion getestet wurde der Versuch mit künstlich gehemmter Thrombozytenfunktion wiederholt wird, um das Ergebnis zu verifizieren.

Nach dem Verfahren werden die Thrombozyten in einem nicht aktivierten Probenfluid, das auf einen Schwingquarz aufgebracht wird aufgrund des Abfalls von Schwingungsparametern als adhäsionsfähig eingestuft.

Um die Aggregationsfähigkeit der Thrombozyten im Probenfluid zu untersuchen, muss dieses aktiviert werden. Die Aktivierung der Probe erfolgt durch Zugabe eines Aktivators. Der Aktivator kann entweder kurz vor der Applikation der Probe eingebracht werden oder der Schwingquarz selbst weist an seiner Oberfläche einen bereits eingebrachten Aktivator auf. Entsprechend dem Verfahren wird auch hier der Schwingungsparameter über die Zeit aufgezeichnet. Stellt man einen sehr starken Abfall der Schwingungsparameter etwa in exponentieller Form fest, werden die Thrombozyten als aggregationsfähig eingestuft.

Darüber hinaus kann durch Zugabe verschiedener Aktivatoren ermittelt werden, welcher Rezeptor der Thrombozyten eine Dysfunktion aufweist. Die unterschiedlichen Aktivierungszweige können somit über Zugabe von entweder van Willebrand Faktor (vWF), Archidonsäure (AA), Adenosin-Diphosphat (ADP) oder Fibrinogen ausgelöst werden, was eine Eingrenzung der Dysfunktion der entsprechenden Aktivierungszweige erlaubt.

Diese Eingrenzung kann auch dadurch erfolgen, dass bei einer erfolgreichen Aggregation der Thrombozyten unterschiedliche Rezeptoren blockiert werden. Sind alle Rezeptoren bis auf einen zu untersuchenden Rezeptor blockiert, kann festgestellt werden, ob dieser Rezeptor eine Störung aufweist, indem das Aggregationsverhalten der aktivierten Probe gemessen wird und man bei einem Verlauf der Schwingungsparameter, der einer Adhäsion entspricht, auf eine Störung des nicht blockierten Rezeptors schließen kann.

Insbesondere durch Aktivierung und Blockierung einzelner Rezeptoren werden jene identifiziert, welche Funktionsstörungen aufweisen. Des Weiteren ist die Blockierung oder Aktivierung einzelner Gruppen von Rezeptoren durch einen spezifischen Antikörper oder Teile davon vorgesehen. Darüber hinaus kann diese Funktion auch durch andere spezifisch bindende Moleküle wie zum Beispiel Lektine oder Fragmente von Nukleinsäuren bewirkt werden.

Vorteilhafte Auswirkungen hat es auch, einen Aktivator, wie beispielsweise Fibrinogen bereits in die Oberfläche beispielsweise eines Schwingquarzes zu integrieren. Man umgeht dabei das Problem, dass der Zeitpunkt von der Aktivierung der Probe bis zur Messung nicht besonders kurz sein muss und daher die gesamte Vorrichtung einfacher gestaltet werden kann. Die Thrombozyten adhärieren an der Fibrinogenschicht und werden gleichzeitig aktiviert, wodurch die Aggregation ausgelöst wird.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: einen Verfahrensablauf zur Bestimmung der Thrombozytenfunktion, und
- Fig. 2: schematische Darstellung des Vorlaufs von Schwingungsparametern für Dysfunktion und Adhäsion und Aggregation.

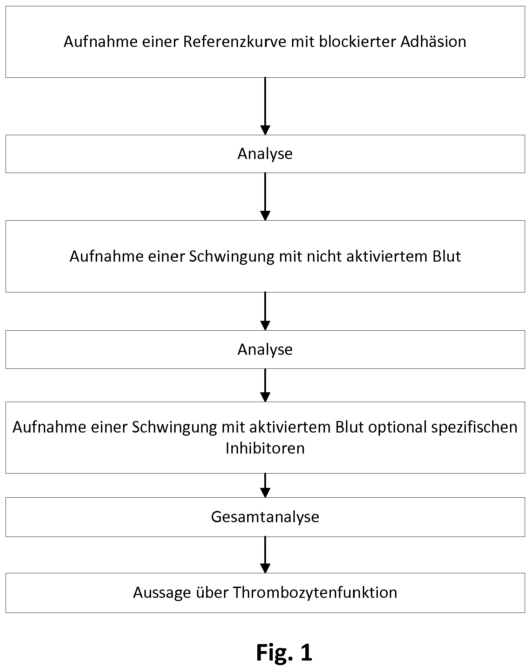

In Fig.1 ist der Ablauf zur Untersuchung der Thrombozytenfunktion einer Blutprobe dargestellt. Als erstes werden Thrombozyten, die nicht aktiviert sind und deren Adhäsionsfähigkeit blockiert ist auf einen Schwingquarz aufgebracht. Die Adhäsionsfähigkeit kann künstlich gestört werden, indem beispielsweise der Collagenrezeptor oder GPIb-IX Rezeptor durch Abciximab blockiert wird. Dies verhindert die Adhäsion der Thrombozyten auf der Schwingquarzoberfläche, wodurch eine unbelastete Referenzkurve der Resonanzfrequenz aufgenommen wird.

Nach Aufnahme der Referenzkurve wird eine weiteres Probenfluid entweder auf denselben Schwingquarz oder auf den gereinigten Schwingquarz aufgebracht. Dieses Probenfluid enthält Thrombozyten deren Rezeptoren bezüglich der Adhäsion weder blockiert noch aktiviert sind. Anschließend erfolgt die Analyse der Kurve. Fällt die Resonanzfrequenz über die Zeit stärker ab, als dies bei der Probe mit den gehemmten Adhäsionsrezeptoren der Fall war, sind die Thrombozyten in dem Probenfluid adhäsionsfähig und es schließt sich ein weiterer Messschritt an.

In einem weiteren Schritt wird eine mit einem Aktivator induziertes Probenfluid beispielsweise mit Adenosin-Diphosphat (ADP), van Willebrand Faktor (vWF) oder einem anderen Faktor auf die Schwingquarzoberfläche aufgebracht. Fällt die gemessene Resonanzfrequenz über die Zeit stärker ab als im vorangegangenen Schritt, in dem die Adhäsion gemessen wurde, lässt sich dann bei einer Aktivierung durch Fibrinogen darauf schließen, dass die primäre Aggregation funktionsfähig ist. In einem weiteren Schritt wird ein Aktivator oder eine mit einem Aktivator induzierte Probe zugegeben, was die sekundäre Aggregation auslöst.

Nimmt die Resonanzfrequenz über die Zeit deutlich stärker ab als zuvor, ist auch die sekundäre Aggregation funktionsfähig. Beeinflussen entsprechend zugegebene Aktivatoren das Schwingungsverhalten des Resonators nicht, wird auf eine Fehlfunktion der korrespondierenden Rezeptoren geschlossen.

Durch diesen Ablauf ist ein grundsätzlicher Ablauf gegeben, der darin besteht, zuerst eine inhibierte, dann eine nicht-aktivierte und anschließend eine aktivierte Probe, unterbrochen durch beliebige Spülschritte, zu vermessen. Darüber hinaus können auf diese Weise mehrere Kombinationen aus Inhibition und Aktivierung in diesen Ablauf aufgenommen werden.

Auf diese Weise lässt sich eindeutig der gestörte Zweig der zellulär vermittelten Gerinnung ermitteln.

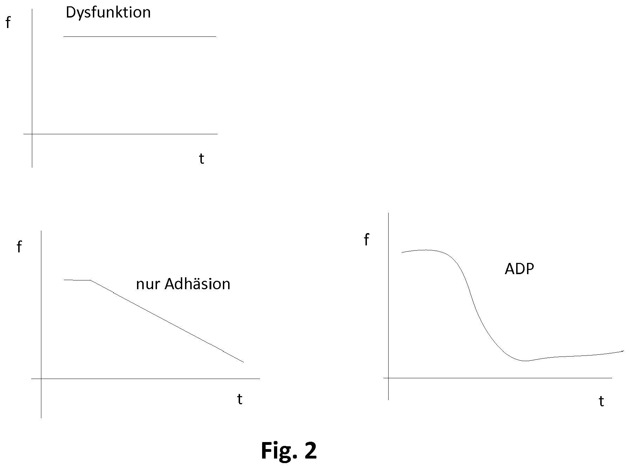

Fig. 2 zeigt drei Frequenzverläufe des Schwingquarzes über die Zeit von drei unterschiedlichen Thrombozytenfunktionen. Der erste Frequenzverlauf zeigte eine gerade Kurve über die gesamte Zeit.

Ein Frequenzabfall ist in diesem Fall nicht festzustellen. Dies bedeutet, dass sich keine oder vernachlässigbar wenige Thrombozyten an der Schwingquarzoberfläche anlagern. Ein solcher Frequenzverlauf lässt eindeutig auf eine Dysfunktion der Thrombozyten schließen. Möglicherweise ist dieser auch durch eine bewusste Blockade durch Zugabe von Abciximab daran gehindert, sich an der Schwingquarzoberfläche anzulagern. Die Thrombozyten sind nicht in der Lage zu adhärieren, entweder aufgrund einer Dysfunktion oder weil diese beispielsweise mit Abciximab bewusst gehemmt wurden.

Die zweite Kurve zeigt einen linear abfallenden Verlauf der Frequenz über die Zeit. Dies zeigt, dass sich nach und nach eine zunehmende Anzahl von Thrombozyten und andere Blutbestandteile an der Schwingquarzoberfläche anlagern. Ein derartiger Frequenzverlauf lässt den eindeutigen Schluss zu, dass eine Adhäsion stattfindet.

In einem dritten Graph ist eine stark abfallende Frequenzkurve dargestellt. Ein solcher Kurvenverlauf kann lässt auf das Vorhandensein funktionsfähiger Thrombozyten schließen, die mindestens einen funktionsfähigen Rezeptor für die Aggregation aufweisen. Für eine Aggregation muss das Blut aktiviert werden. Dies kann mittels Adenosin-Diphosphat (ADP), Fibrinogen oder dem van Willebrand Faktor (vWF) ausgelöst werden. Möglich ist auch eine Aktivierung mittels Archidonsäure (AA). Abhängig vom verwendeten Aktivator beziehungsweise einem etwaig angewandten selektiven Inhibitor kann so die Funktionsfähigkeit der einzelnen Rezeptoren der Thrombozyten überprüft werden.

## Patentansprüche

1. Verfahren zur Bestimmung von Parametern der Hämostase mittels der Messung von Schwingungsparametern eines Resonators mit einer Messoberfläche, die in Kontakt mit einem Thrombozyten enthaltenden Probenfluid steht, wobei als Schwingungsparameter die Resonanzfrequenz, die Amplitude und / oder die Dämpfung über die Zeit aufgezeichnet wird und so ein Verlauf des Schwingungsparameters über die Zeit erstellt wird, wobei das Probenfluid aktiviert wird, **dadurch gekennzeichnet, dass** die Charakteristik des Verlaufs des Schwingungsparameters über die Zeit ausgewertet wird, wobei die Thrombozytenfunktion derart bewertet wird, dass wenn das Schwingungsverhalten des Schwingquarzes mit aktiviertem Probefluid dem mit einem nicht aktivierten Probenfluid entspricht, darauf geschlossen wird, dass die Thrombozyten adhäsionsfähig aber nicht aggregationsfähig sind also eine Aggregationsstörung vorliegt, und dass bei einem zeitlich unveränderten Verlauf des Schwingungsparameters eine Adhäsionsstörung diagnostiziert wird.

2. Verfahren nach Anspruch 1, **dadurch gekennzeichnet, dass** die Thrombozytenfunktion durch Zugabe verschiedener Aktivatoren, die unterschiedliche Aktivierungszweige auslösen, näher spezifiziert wird.

3. Verfahren nach einem der vorangegangenen Ansprüche, **dadurch gekennzeichnet, dass** das Aktivierungszweige durch Zugabe entsprechender Inhibitoren blockiert werden.

4. Verfahren nach den Ansprüchen 2 oder 3, **dadurch gekennzeichnet, dass** durch Aktivierung und Blockierung einzelner Rezeptoren diejenigen identifiziert werden, die Funktionsstörungen aufweisen.

5. Verfahren nach einem der vorangegangenen Ansprüche, **dadurch gekennzeichnet, dass** die Blockierung oder Aktivierung einzelner oder Gruppen von Rezeptoren durch einen spezifischen Antikörper, durch Teile davon oder durch andere spezifisch bindende Moleküle wie Lektine, Fragmente von Nukleinsäuren oder Ähnlichem bewirkt wird.

## Claims

1. Method for determining haemostasis parameters by measuring vibration parameters of a resonator having a measuring surface which contacts a platelet containing sample fluid, wherein the resonance frequency, the amplitude and/or the damping is recorded over time and used as the vibration parameter, thus providing a curve of the vibration parameter over time, wherein the sample fluid is activated, **characterized in that** the characteristics of the curve of the vibration parameter are evaluated over time and the platelet function is then assessed as follows: when the vibration behaviour of the crystal resonator with an activated sample fluid corresponds to the vibration behaviour of a crystal resonator with a non-activated sample fluid, it is concluded that the platelets are capable of adhesion but are incapable of aggregation, i.e. that there is an aggregation disorder, and when the curve of the vibration parameter remains unchanged over time, an adhesion disorder is diagnosed.

2. Method according to claim 1, **characterized in that** the platelet function is further specified by adding different activators which trigger different activation branches.

3. Method according to one of the preceding claims, **characterized in that** the activation branches are blocked by the addition of suitable inhibitors.

4. Method according to claims 2 or 3, **characterized in that** activating and blocking individual receptors will allow identification of those that have functional defects.

5. Method according to one of the preceding claims, **characterized in that** blocking or activating individual receptors or receptor groups is achieved by means of a specific antibody, parts thereof or other specifically binding molecules such as lectins, nucleic acid fragments or the like.

## Revendications

1. Procédé de détermination des paramètres de l'hémostase au moyen de la mesure de paramètres d'oscillation d'un résonateur ayant une surface de mesure qui est en contact avec un fluide d'échantillon contenant des thrombocytes, dans lequel la fréquence de résonance, l'amplitude et/ou l'évaporation sur la durée est enregistrés comme paramètre d'oscillation et une courbe du paramètre d'oscillation sur la durée est ainsi établie, dans lequel le fluide d'échantillon est activé, **caractérisé en ce que** la caractéristique de la courbe du paramètre d'oscillation sur la durée est évaluée, dans lequel la fonction des thrombocytes est appréciée de telle sorte que quand le comportement d'oscillation du quartz oscillant avec le fluide d'échantillon activé correspond à un fluide d'échantillon non activé, on en déduit que les thrombocytes sont capables d'adhésion mais pas d'agrégation, qu'il existe donc un trouble de l'agrégation et que, pour une courbe du paramètre d'oscillation, non modifiée dans la durée, un trouble de l'adhésion est diagnostiqué.

2. Procédé selon la revendication 1, **caractérisé en ce que** la fonction des thrombocytes est spécifiée plus en détail par addition de divers activateurs qui déclenchent différentes branches d'activation.

3. Procédé selon l'une des revendications précédentes, **caractérisé en ce que** la branche d'activation est bloquée par l'addition d'inhibiteurs correspondants.

4. Procédé selon les revendications 2 ou 3, **caractérisé en ce que** l'on identifie des récepteurs individuels qui présentent des dysfonctionnements par activation et par blocage.

5. Procédé selon l'une des revendications précédentes, **caractérisé en ce que** le blocage ou l'activation des différents récepteurs ou groupes de récepteurs par un anticorps spécifique est provoqué par des fractions de ceux-ci ou d'autre molécules à liaison spécifique comme les lectines, les fragments d'acides nucléiques ou similaires.
